# EUROPEAN PATENT APPLICATION

(11) **EP 3 689 340 A1**
(43) Date of publication of application: **05.08.2020**
(21) Application number: 18860618.0
(22) Date of filing: 27.09.2018
(51) Int. Cl.: A61K 31/135, A61P 25/16, A61P 25/28

(54) **R-KETAMINE AND DERIVATIVE THEREOF AS PROPHYLACTIC OR THERAPEUTIC AGENT FOR NEURODEGENERATION DISEASE OR RECOGNITION FUNCTIONAL DISORDER**

(30) Priority: 27.09.2017 JP 2017186867
(71) Applicant: National University Corporation Chiba University, Chiba-shi, Chiba 267-8522 (JP)
(72) Inventor: HASHIMOTO, Kenji, Chiba-shi Chiba 260-8670 (JP)
(74) Representative: Cooley (UK) LLP
(86) International application number: PCT/JP2018/036079
(87) International publication number: WO 2019/065900

(57) **Abstract**

The present invention is to provide a preventive or therapeutic agent for a neurodegenerative disease or a cognitive dysfunction, containing a compound represented by a following formula (I) or a pharmacologically acceptable salt thereof as an active ingredient.

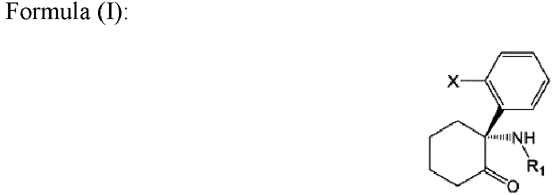

## Description

### [Technical Field]

The present invention relates to a preventive or therapeutic agent for a neurodegenerative disease or a cognitive dysfunction, which contains R-ketamine and a derivative thereof.

### [Background Art]

As aging of society progresses, the number of cases of neurodegenerative diseases such as Parkinson's disease, Alzheimer's disease, and Lewy body dementia has been increasing, and the development of a preventive or therapeutic agent for these neurodegenerative diseases is urgently needed.

At present, for the treatment of Parkinson's disease, an L-dopa formulation, a dopamine release promotor, a dopaminergic stimulant, a catechol-O-methyltransferase (COMT) inhibitor, a monoamine oxidase B (MAOB) inhibitor, and the like are used, and treatment is essentially a dopamine replacement therapy.

For the treatment of dementia such as Alzheimer's disease, donepezil hydrochloride, galantamine, and rivastigmine, which are inhibitors of the enzyme cholinesterase that degrades acetylcholine, and memantine, a blocker of the N-methyl-D-aspartic acid (hereinafter, abbreviated as NMDA) receptor which is a glutamate receptor, have been used.

However, these existing therapeutic agents cannot suppress neuronal damage and loss, and thus they cannot stop the progression of symptoms. In addition, it is difficult to say that these existing therapeutic agents have a sufficient therapeutic effect on these neurodegenerative diseases, and currently, there are practically no effective preventive methods or therapeutic methods.

For example, when L-dopa is used for treating Parkinson's disease, while the effect of L-dopa is still there, the symptoms of Parkinson's disease can be suppressed, and the patient can live normally. However, when L-dopa is no longer effective, the symptoms of Parkinson's disease appear. This is the on-off phenomenon of Parkinson's disease, and this has become a major problem for dopamine replacement therapy in clinical settings.

Positron emission tomography (PET) studies have shown that β-amyloid is deposited in the brains of Alzheimer's disease patients. However, a number of new drug candidates (β-amyloid antibodies and the like) based on the β-amyloid hypothesis of Alzheimer's disease have failed in clinical trials (non-patent literature 1).

In these neurodegenerative diseases, the loss of nerve cells in the brain or spinal cord is observed as the symptoms worsen. For example, in the brain of patients who died from Parkinson's disease, marked loss of dopaminergic neurons of the nigrostriatal system is observed, and in the brain of patients who died from Alzheimer's disease, loss of acetylcholine nerve and deposition of β-amyloid and tau protein are observed in a number of brain regions including the frontal cortex and the hippocampus. When considering the importance of neural loss and abnormal protein deposition in the pathogenesis of these neurodegenerative diseases, compounds with a neuroprotective effect and a neurotrophic effect are considered to be preventive or therapeutic agents for these neurodegenerative diseases.

It has been reported that RS-ketamine, which is known to be a NMDA receptor antagonist, has a neuroprotective effect in an animal model of Parkinson's disease (non-patent literature 2). However, in non-patent literature 2, RS-ketamine is used at a high dose of 50 mg/kg, which is an anesthetic dose. At this dose, side effects such as an acute activity increase effect are notably observed.

In addition, the neuroprotective effect of RS-ketamine in an animal model of Parkinson's disease under anesthesia has been studied by using RS-ketamine in combination with xylazine, which is an anesthetic (non-patent literature 3).

It should be noted that although it has been suggested that glutamate neurotransmission via the NMDA receptor is involved in the pathogenesis of Parkinson's disease, since RS-ketamine is designated as a narcotic because it has problems of side effects related to mental symptoms such as hallucinations and delusions, dissociation symptoms, and dependence (non-patent literature 4), there is difficulty in clinical implementation.

In addition, among RS-ketamines, it is known that S-ketamine, which is the S-isomer, has an affinity for the NMDA receptor about four times higher than R-ketamine, which is the R-isomer (non-patent literature 4).

On the other hand, it has been reported that R-ketamine has fewer side effects (psychotic disorder triggering effect, drug dependency, and the like) as compared with S-ketamine in animal experiments, and the intensity of the psychotic disorder symptom triggering effect of ketamine correlates to the blocking intensity of the NMDA receptor (non-patent literature 5).

Furthermore, it is generally understood that both the analgesic effect and the psychotic disorder symptom triggering effect of RS-ketamine are mainly mediated by blockade of the NMDA receptor (non-patent literature 6). It is believed that the effect of RS-ketamine is mainly caused by S-ketamine because the affinity of S-ketamine for the NMDA receptor is high.

Cognitive dysfunction is observed not only in psychiatric diseases such as bipolar disorder, major depressive disorder, general anxiety disorder, panic disorder, obsessive compulsive disorder (OCD), post-traumatic stress disorder (PTSD), attention deficit hyperactivity disorder (ADHD), autism spectrum disorder (ASD), eating disorders, and substance use disorders (drug dependency) (non-patent literature 7), but also in the neurodegenerative diseases described above and a number of medical diseases (cardiovascular diseases, cancer (also referred to as malignant tumors), and the like) (non-patent literatures 7 to 12). However, there is no drug for preventing or treating these cognitive dysfunctions.

### [Prior Art Literature]

### [Non-patent Literature]

[Non-patent Literature 1] Abbott A. et al. Failed Alzheimer's trial does not kill leading theory of disease. Nature. 2016 Nov 23; 540 (7631): 15-16.
[Non-patent Literature 2] Pan J. et al. Blockade of the translocation and activation of c-Jun N-terminal kinase 3 (JNK3) attenuates dopaminergic neuronal damage in mouse model of Parkinson's disease. Neurochem Int. 2009 Jun; 54 (7): 418-25.
[Non-patent Literature 3] Ferro1 MM. et al. Neuroprotective effect of ketamine/xylazine on two rat models of Parkinson's disease. Braz J Med Biol Res. 2007 Jan; 40 (1): 89-96.
[Non-patent Literature 4] Domino EF. Taming the ketamine tiger. 1965. Anesthesiology. 2010 Sep; 113 (3): 678-84.
[Non-patent Literature 5] Yang C. et al. Loss of parvalbumin-immunoreactivity in mouse brain regions after repeated intermittent administration of esketamine, but not R-ketamine. Psychiatry Res. 2016 May 30; 239: 281-3.
[Non-patent Literature 6] Javitt DC. et al. Recent advances in the phencyclidine model of schizophrenia. Am J Psychiatry. 1991 Oct; 148 (10): 1301-8.
[Non-patent Literature 7] Millan MJ, et al. Cognitive dysfunction in psychiatric disorders: characteristics, causes and the quest for improved therapy. Nat Rev. Drug Discov. 2012 Feb 1; 11 (2): 141-168.
[Non-patent Literature 8] Hachinski V. et al. National Institute of Neurological Disorders and Stroke-Canadian Stroke Network vascular cognitive impairment harmonization standards. Stroke. 2006 Sep; 37 (9): 2220-41.
[Non-patent Literature 9] Lopez OL. et al. Risk factors for mild cognitive impairment in the Cardiovascular Health Study Cognition Study: part 2. Arch Neurol. 2003 Oct; 60 (10): 1394-9.
[Non-patent Literature 10] Tannock IF et al. Cognitive impairment associated with chemotherapy for cancer: report of a workshop. J Clin Oncol. 2004 Jun 1; 22 (11): 2233-9.
[Non-patent Literature 11] van Dam FS et al. Impairment of cognitive function in women receiving adjuvant treatment for high-risk breast cancer: high-dose versus standard-dose chemotherapy. J Natl Cancer Inst. 1998 Feb 4; 90 (3): 210-8.
[Non-patent Literature 12] Vogels RL et al. Cognitive impairment in heart failure: a systematic review of the literature. Eur J Heart Fail. 2007 May; 9 (5): 440-9.

### [Summary of Invention]

### [Problem to be Solved by Invention]

Non-patent literatures 2 and 3 both show the neuroprotective effect of RS-ketamine under anesthesia. Since prevention or treatment of neurodegenerative disease may not be carried out for patients under anesthesia, it is difficult to say that the disclosures in non-patent literatures 2 and 3 have confirmed that RS-ketamine is clinically effective for Parkinson's disease.

The problem to be solved by the present invention is to provide a new compound having a preventive or therapeutic effect on a neurodegenerative disease or a cognitive dysfunction.

### [Means for Solving Problem]

The present inventors have carried out intensive studies to solve the problems described above, found a new compound having a preventive or therapeutic effect on a neurodegenerative disease or a cognitive dysfunction, and completed the present invention.

That is, the present invention is as follows.
(1) A preventive or therapeutic agent for a neurodegenerative disease or a cognitive dysfunction, containing a compound represented by the following formula (I) or a pharmacologically acceptable salt thereof as an active ingredient.
   Formula (I): 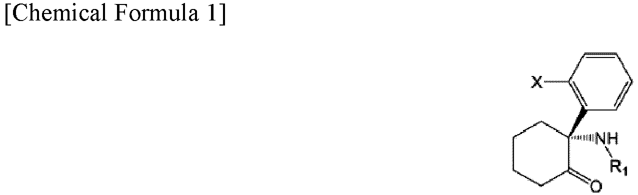 (In the formula (I),
   X is H, alkyl, alkenyl, aryl, F, Cl, Br, or I; and
   Ri is alkyl, alkenyl, or aryl.)
(2) The preventive or therapeutic agent for a neurodegenerative disease or a cognitive dysfunction according to (1), containing R-ketamine or a pharmacologically acceptable salt thereof.
(3) The preventive or therapeutic agent for a neurodegenerative disease or a cognitive dysfunction according to (1) or (2), wherein the neurodegenerative disease includes Parkinson's disease, Parkinson's syndrome (multiple system atrophy, progressive supranuclear palsy, corticobasal degeneration, and the like), a disease that shows chorea movement (Huntington's disease, spiny erythrocyte chorea, and the like), spinal cord cerebellar degeneration, amyotrophic lateral sclerosis, spinal muscular atrophy, primary lateral sclerosis, spinal and bulbar muscular atrophy, syringomyelia, neurospinous erythrocytosis, eating disorders, substance use disorders (drug dependency), Alzheimer's disease, Lewy body dementia, basal ganglia degeneration, multiple sclerosis, or the like or includes a neurodegenerative disease based on traumatic brain injury, cerebral infarction, or cardiovascular disease, preferably Parkinson's disease, Parkinson's syndrome, Alzheimer's disease, or Lewy body dementia, and more preferably Parkinson's disease, Alzheimer's disease, or Lewy body dementia.
   Here, the present invention may be a preventive or therapeutic agent for eating disorders or substance use disorders (drug dependency), containing a compound represented by the following formula (I) or a pharmacologically acceptable salt thereof as an active ingredient.
(4) The preventive or therapeutic agent for a neurodegenerative disease or a cognitive dysfunction according to (1) or (2), wherein the cognitive dysfunction is a cognitive dysfunction based on a neurodegenerative disease or a cognitive dysfunction based on a psychiatric disease including eating disorders, substance use disorders (drug dependency), depression, bipolar disorder, anxiety disorders, and autism, a circulatory disease, and a medical disease including cancer, preferably cognitive dysfunction based on neurodegenerative disease, and wherein preferable specific cognitive dysfunctions include a cognitive dysfunction based on Parkinson's disease, Parkinson's syndrome, Alzheimer's disease, Lewy body dementia, or substance use disorders (drug dependency).
   Here, the neurodegenerative disease in the cognitive dysfunction based on a neurodegenerative disease may be the neurodegenerative disease according to (3).
(5) The preventive or therapeutic agent for a neurodegenerative disease or a cognitive dysfunction according to any one of (1) to (4), not substantially containing a compound represented by a following formula (II) or a pharmacologically acceptable salt thereof.
   Formula (II): 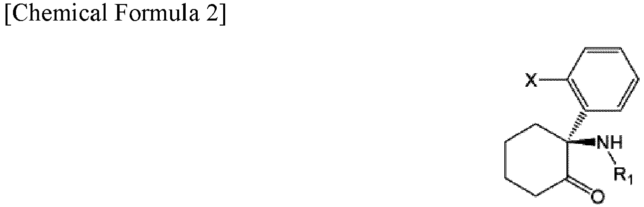 (In the formula (II),
   the definitions of X and Ri are the same as the definitions in the formula (I).)
(6) The preventive or therapeutic agent for a neurodegenerative disease or a cognitive dysfunction according to any one of (1) to (5), not substantially containing S-ketamine or a pharmacologically acceptable salt thereof.
(7) A preventive or therapeutic agent for a neurodegenerative disease or a cognitive dysfunction composed of a compound represented by the formula (I) described above or a pharmacologically acceptable salt thereof as an active ingredient.
(8) A preventive or therapeutic agent for a neurodegenerative disease or a cognitive dysfunction composed of R-ketamine or a pharmacologically acceptable salt thereof.
(9) A pharmaceutical composition containing the preventing or therapeutic agent for a neurodegenerative disease or a cognitive dysfunction according to any one of (1) to (7).
(10) A pharmaceutical composition for preventing or treating a neurodegenerative disease or a cognitive dysfunction, containing a compound represented by the formula (I) described above or a pharmacologically acceptable salt thereof in an amount effective for reducing the symptoms of a neurodegenerative disease or a cognitive dysfunction and not substantially containing the compound represented by the formula (II) described above or a pharmacologically acceptable salt thereof.
(11) A pharmaceutical composition for preventing or treating a neurodegenerative disease or a cognitive dysfunction, containing R-ketamine or a pharmacologically acceptable salt thereof in an amount effective for reducing the symptoms of a neurodegenerative disease or a cognitive dysfunction and not substantially containing S-ketamine or a pharmacologically acceptable salt thereof.
(12) A compound represented by the formula (I) described above or a pharmacologically acceptable salt thereof for preventing or treating a neurodegenerative disease or a cognitive dysfunction.
(13) R-ketamine or a pharmacologically acceptable salt thereof for preventing or treating a neurodegenerative disease or a cognitive dysfunction.
(14) A method for preventing or treating a neurodegenerative disease or a cognitive dysfunction, wherein a compound represented by the formula (I) described above or a pharmacologically acceptable salt thereof is administered to a patient in need thereof.
(15) A method for preventing or treating a neurodegenerative disease or a cognitive dysfunction, wherein R-ketamine or a pharmacologically acceptable salt thereof is administered to a patient in need thereof.

### [Effect of Invention]

A compound represented by the formula (I) typified by R-ketamine or a pharmacologically acceptable salt thereof is effective for preventing or treating a neurodegenerative disease or a cognitive dysfunction.

### [Brief Description of Drawings]

FIG. 1 is a diagram describing a test plan for studying the effects of R-ketamine and S-ketamine in a model animal of Parkinson's disease.
FIG. 2 is a representative DAT immunohistochemical photograph of groups (A) to (D) in MPTP-treated mice. (A) shows a physiological saline + physiological saline administration group, (B) shows an MPTP + physiological saline administration group, (C) shows an MPTP + R-ketamine administration group, and (D) shows an MPTP + S-ketamine administration group. The shade of the photograph indicates the density of the DAT protein. DAT means dopamine transporter.
FIG. 3 shows the DAT density data of groups (A) to (D) in the MPTP-treated mice.
FIG. 4 is a diagram describing a test plan for studying the effect of a single administration of R-ketamine in a model animal of Parkinson's disease.
FIG. 5 shows the DAT density data of three groups in the MPTP-treated mice. The physiological saline + physiological saline administration group, the MPTP + physiological saline administration group, and the MPTP + R-ketamine administration group are shown.
FIG. 6 is a diagram describing a test plan for studying the effect of R-norketamine and S-norketamine administration in a model animal of Parkinson's disease.
FIG. 7 shows the DAT density data of four groups in the MPTP-treated mice. The physiological saline + physiological saline administration group, the MPTP + physiological saline administration group, the MPTP + R-norketamine administration group, and the MPTP + S-norketamine administration group are shown.
FIG. 8 is a diagram describing a test plan for examining the effect of ANA-12 on the therapeutic effect of R-ketamine in a model animal of Parkinson's disease.
FIG. 9 shows the DAT density data of five groups in the MPTP-treated mice. The physiological saline + physiological saline administration group, the MPTP + physiological saline administration group, the MPTP + R-ketamine administration group, the MPTP + R-ketamine/ANA-12 administration group, and the MPTP + ANA-12 administration group are shown.
FIG. 10 is a diagram describing a test plan for studying the effects of R-ketamine and S-ketamine in a model animal of Alzheimer's disease.
FIG. 11 shows the data of a novel object recognition test (NORT: Novel Object Recognition Test) of four groups in the β-amyloid-treated mice. The physiological saline + physiological saline administration group, the
β-amyloid + physiological saline administration group, the β-amyloid + R-ketamine administration group, and the β-amyloid + S-ketamine administration group are shown.
FIG. 12 is a diagram describing a test plan for studying the effect of R-ketamine in a model animal of Lewy body dementia or the like.
FIG. 13 shows the data of a novel object recognition tests (NORT) of three groups in the α-synuclein-treated mice. The physiological saline + physiological saline administration group, the α-synuclein + physiological saline administration group, and the α-synuclein + R-ketamine administration group are shown.

### [Examples of Invention]

The preventive or therapeutic agent for a neurodegenerative disease or a cognitive dysfunction of the present invention contains a compound represented by the following formula (I) or a pharmacologically acceptable salt thereof as an active ingredient (hereinafter, the compound represented by the formula (I) may be simply referred to as "compound I" in some cases.).
Formula (I): 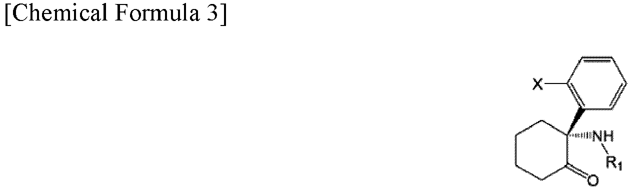 (In the formula (I),
X is H, alkyl, alkenyl, aryl, F, Cl, Br, or I; and
Ri is alkyl, alkenyl, or aryl.)
X is H, alkyl, alkenyl, aryl, F, Cl, Br, or I, preferably F or Cl, and more preferably Cl.
Ri is alkyl, alkenyl, or aryl.

In the present invention, the number of carbon atoms of alkyl and alkenyl is preferably 1 to 10, more preferably 1 to 6, and still more preferably 1 to 5.

When X and Ri are alkyl, there is no particular limitation, but examples thereof include methyl, ethyl, propyl, isopropyl, cyclopropyl, butyl, isobutyl, t-butyl, cyclobutyl, cyclopropylmethyl, pentyl, cyclopentyl and the like. It is preferably a linear alkyl, more preferably a linear chain alkyl having one to five carbon atoms, even more preferably methyl or ethyl, and still more preferably methyl.

When X and Ri are alkenyl, they may be an alkenyl which is a group having one or more double bonds in the group listed for alkyl. Although there is no particular limitation, examples thereof include vinyl, allyl, and the like.

When X and Ri are aryl, although there is no particular limitation, examples thereof include phenyl and the like.

In the formula (I), the compound wherein X is Cl and Ri is methyl is R-ketamine.

The preventive or therapeutic agent for a neurodegenerative disease or a cognitive dysfunction according to the present invention preferably contains R-ketamine or a pharmacologically acceptable salt thereof as an active ingredient.

The present invention relates to a preventive or therapeutic agent for a neurodegenerative disease or a cognitive dysfunction, containing the compound I or a pharmacologically acceptable salt thereof. Furthermore, the present invention relates to a pharmaceutical composition for preventing or treating a neurodegenerative disease or a cognitive dysfunction, containing the compound (I) or a pharmacologically acceptable salt thereof in an amount effective for reducing the symptoms of a neurodegenerative disease or a cognitive dysfunction and not substantially containing the compound represented by the formula (II) or a pharmacologically acceptable salt thereof (hereinafter, the compound represented by the formula (II) may be simply referred to as "compound II" in some cases.).
Formula (II): 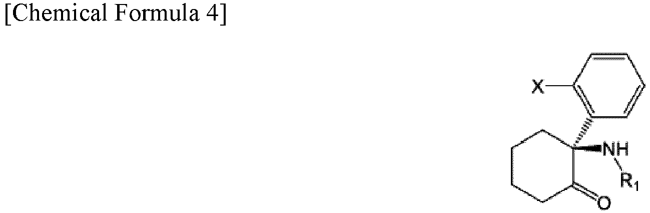 (In the formula (II),
the definitions of X and Ri are the same as the definitions in the formula (I).)

In the present invention, the term "as an active ingredient" means to contain as a main active ingredient, and if the compound I or a pharmacologically acceptable salt thereof is contained as a medical ingredient, the content thereof is not particularly limited.

When the compound I or a pharmacologically acceptable salt thereof is contained as an active ingredient, it is preferable that the compound II or a pharmacologically acceptable salt thereof is not substantially contained.

In the present invention, the term "not substantially containing the compound represented by the formula (II) or a pharmacologically acceptable salt thereof' means that the compound II or a pharmacologically acceptable salt thereof is not contained at all, the compound II or a pharmacologically acceptable salt thereof may be contained in such an amount that the effect or side effect does not occur, or it may be contained as an impurity inevitably mixed in the production.

In addition, the term "not substantially containing the compound represented by the formula (II) or a pharmacologically acceptable salt thereof' may mean that "the compound represented by the formula (II) or a pharmacologically acceptable salt thereof' may be contained as a impurity as in the "Impurities in New Drug Substances" for the compound represented by the formula (I) which is a drug substance in the case of a drug or a pharmacologically acceptable salt thereof, and the drug substance may contain 0.15% or less of the compound II.

In the present invention, "the compound represented by the formula (I)" in "the compound represented by the formula (I) or a pharmacologically acceptable salt thereof' is preferably R-ketamine, and "the compound represented by the formula (II)" in "the compound represented by the formula (II) or a pharmacologically acceptable salt thereof' is preferably S-ketamine.

It should be noted that RS-ketamine is a racemic mixture which is an equal mixture of R-ketamine and S-ketamine.

In the present invention, the term "neurodegenerative disease" means a disease, in which a specific group of nerve cells among nerve cells in the brain and spinal cord, is gradually damaged and lost.

The neurodegenerative disease is not particularly limited, but examples thereof include Parkinson's disease, Parkinson's syndrome, multiple system atrophy, progressive supranuclear palsy, corticobasal degeneration, and the like, which affect movement, spinal cord cerebellar degeneration and the like, which affect body balance, amyotrophic lateral sclerosis and the like, which cause muscle weakness, and Alzheimer's disease, Lewy body dementia, basal ganglia degeneration, and the like, in which cognitive function is decreased. In addition, R-ketamine having a neuroprotective effect may be used for the treatment of a disease associated with a decrease in a substance like a neurotrophic factor, that is, neurodegeneration (loss of nerve cells). Such neurodegenerative disease is not particularly limited, but examples thereof include Parkinson's disease, Parkinson's syndrome (multiple system atrophy, progressive supranuclear palsy, corticobasal degeneration, and the like), a disease that shows chorea movement (Huntington's disease, spiny erythrocyte chorea, and the like), spinal cord cerebellar degeneration, amyotrophic lateral sclerosis, spinal muscular atrophy, primary lateral sclerosis, spinal and bulbar muscular atrophy, syringomyelia, neurospinous erythrocytosis, eating disorders, substance use disorders (drug dependency), Alzheimer's disease, Lewy body dementia, basal ganglia degeneration, multiple sclerosis, or the like or include a neurodegenerative disease or the like based on traumatic brain injury, cerebral infarction, or cardiovascular disease.

Preferable examples of neurodegenerative diseases include Parkinson's disease, Parkinson's syndrome, Alzheimer's disease or Lewy body dementia, and more preferably Parkinson's disease, Alzheimer's disease or Lewy body dementia.
The preventive or therapeutic agent for a neurodegenerative disease or a cognitive dysfunction according to the present invention is also suitably used for preventing or treating an eating disorder or a substance use disorder (drug dependency) in an eating disorder patient or a substance use disorder (drug dependency) patient.

In the present invention, the term "cognitive dysfunction" means a state in which it is difficult to memorize something, pay attention to things, take actions based thereon, and carry out actual work. It is known that cognitive dysfunction greatly affects the level of living function more than mental symptoms.

The cognitive dysfunction is not particularly limited, but examples thereof include cognitive dysfunctions based on the neurodegenerative disease described above, cognitive dysfunctions based on psychiatric diseases including eating disorders, substance use disorders, depression, bipolar disorder, anxiety disorder, autism, circulatory diseases, and medical diseases including cancer, and the like.

Psychiatric diseases in cognitive dysfunction based on psychiatric diseases include bipolar disorder, major depressive disorder, general anxiety disorder, panic disorder, obsessive compulsive disorder (OCD), post-traumatic stress disorder (PTSD), attention deficit hyperactivity disorder (ADHD), autism spectrum disorder (ASD), and the like.

A preferable example of cognitive dysfunction is a cognitive dysfunction based on a neurodegenerative disease. In addition, a preferable example of cognitive dysfunction is a cognitive dysfunction based on Parkinson's disease, Parkinson's syndrome, Alzheimer's disease, Lewy body dementia, or substance use disorder (drug dependency).

In the present invention, as shown in the following examples, since Parkinson's disease model animals (Jackson-Lewis V. et al. Protocol for the MPTP mouse model of Parkinson's disease. Nat Protoc. 2007; 2 (1): 141-51).) have brain dopamine nervous system loss and R-ketamine shows a neuroprotective effect on the loss, R-ketamine may be used as a preventive or therapeutic agent for diseases associated with the brain dopamine nervous system loss.
Specifically, examples of diseases associated with brain dopamine loss include cognitive dysfunctions as described above for the description of the term "cognitive dysfunction," and among them, R-ketamine may be used for preventing or treating diseases associated with a decrease of dopamine transporters (DAT). In particular, examples of dysfunctions of the brain dopamine nervous system include substance use disorders (drug dependency) known in abusers of stimulant drugs or cocaine, and R-ketamine is considered to be effective as a preventive or therapeutic drug for substance use disorders (drug dependency). It has been reported that dopamine transporter (DAT) is decreased in the brains of cocaine abusers and stimulant drug (methamphetamine) users, and it has been pointed out that the decrease in DAT is associated with cognitive dysfunction. The following three literatures are cited as references. In addition, the decrease in DAT in substance use disorder patients is similar to the decrease in DAT seen in Parkinson's disease patients.
Sekine Y, et al. Methamphetamine-related psychiatric symptoms and reduced brain dopamine transporters studied with PET. Am J Psychiatry. 2001 Aug; 158 (8): 1206-14.
Volkow ND. et al. Association of dopamine transporter reduction with psychomotor impairment in methamphetamine abusers. Am J Psychiatry. 2001 Mar; 158 (3): 377-82.
Volkow ND. et al. Decreased striatal dopaminergic responsiveness in detoxified cocaine-dependent subjects. Nature. 1997 Apr 24; 386 (6627): 830-3.

In addition, as shown in the following examples, it is considered that the effect of R-ketamine is involved with a signal mediated by the brain-derived neurotrophic factor BDNF. Therefore, R-ketamine is considered to have a BDNF-like neurotrophic factor-like effect, leading to a neuroprotective effect.

Due to these neuroprotective effects, R-ketamine may be used not only for the prevention or treatment of neurodegenerative diseases, but also for the prevention or treatment of cognitive dysfunctions described above as the description of the term "cognitive dysfunction."

By using the preventive or therapeutic agent for a neurodegenerative disease or a cognitive dysfunction according to the present invention, it is possible to prevent the onset of a neurodegenerative disease or a cognitive dysfunction and to exert therapeutic effects such as alleviating and improving symptoms in patients suffering from a neurodegenerative disease or a cognitive dysfunction.

Since a neurodegenerative disease or a cognitive dysfunction is a disease that progresses over a long period (measured in years), early treatment may also prevent the progression of symptoms.

In addition, it may also be used for a patient with a genetic background potentially predisposing him or her to a neurodegenerative disease or a cognitive dysfunction to prevent the onset of a neurodegenerative disease or a cognitive dysfunction by administering it before the symptoms of the neurodegenerative disease or the cognitive dysfunction are exhibited.

In the present invention, since the compound represented by the formula (I) or a pharmacologically acceptable salt thereof is used for the prevention or treatment of a neurodegenerative disease or a cognitive dysfunction, the administration of the drug is scheduled to be a long period of time.

Therefore, by using the compound represented by the formula (I) which is an R-form or a pharmacologically acceptable salt thereof, it is possible to administer the drug for a long period of time without exerting side effects, so the compound represented by the formula (I) or a pharmacologically acceptable salt thereof may be used as a preventive or therapeutic agent for a neurodegenerative disease or a cognitive dysfunction.

The preventive agent for a neurodegenerative disease or a cognitive dysfunction in the present invention may be an agent for preventing the onset of a neurodegenerative disease or a cognitive dysfunction, or an agent for preventing the progression of the symptoms. In addition, the therapeutic agent for a neurodegenerative disease or a cognitive dysfunction according to the present invention has a therapeutic effect of preventing the progression of the symptoms, and alleviating or improving the symptoms.

The compound represented by the formula (I), which is an active ingredient in the present invention, may be used in the form of both a free base and a pharmacologically acceptable salt thereof.

Hereinafter, R-ketamine will be exemplified and described. The items described for R-ketamine can also be applied to the compound represented by the formula (I). In that case, the portion described as S-ketamine may be replaced with the compound represented by the formula (II).

R-ketamine is a free base and has a chemical structure represented by the following formula (III).
Formula (III):

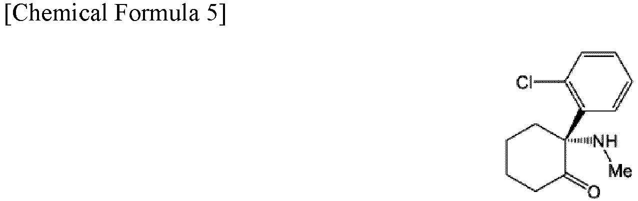

As the pharmacologically acceptable salt of R-ketamine, an addition salt of a pharmacologically acceptable acid is preferable, and the pharmacologically acceptable acid is not particularly limited, but hydrochloric acid is preferable.

R-ketamine hydrochloride has a chemical structure represented by a following formula (IV).
Formula (IV):

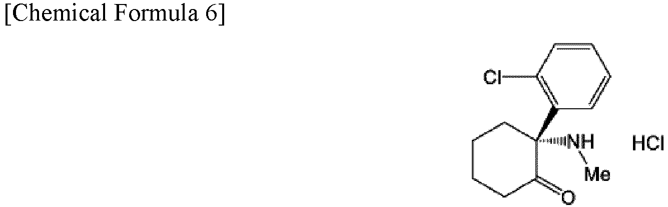

R-ketamine or a pharmacologically acceptable salt thereof used in the present invention may be in the form of a hydrate or in the form of a solvate.

In addition, R-ketamine or a pharmacologically acceptable salt thereof may be labeled with an isotope.

The isotope is not particularly limited, and examples thereof include stable isotopes such as ¹³C and ²H (D).

By carrying out the isotope labeling, the biokinetics of R-ketamine or a pharmacologically acceptable salt thereof before the isotope labeling may be changed. For example, deuterium-labeled R-ketamine hydrochloride having a chemical structure represented by a following formula (V) (in the formula (V), D represents a deuterium atom) is considered to be slowly metabolized, and it is expected that the duration time will be longer.
Formula (V):

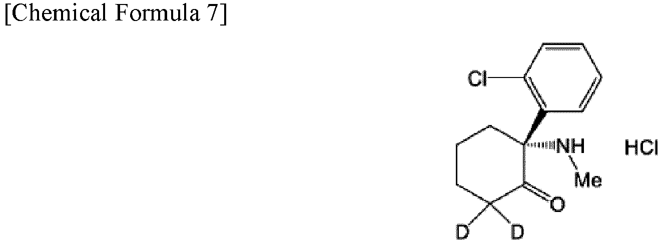

The preventive or therapeutic agent, drug, and pharmaceutical composition for a neurodegenerative disease or a cognitive dysfunction according to the present invention may be administered orally or parenterally.

For oral administration, known dosage forms such as tablets, capsules, coated tablets, troches, liquids such as solutions or suspensions may be used. In addition, examples of parenteral administration include intravenous, intramuscular, or subcutaneous administration by injection, nasal or oral transmucosal administration using powders, drops, sprays, aerosols, or the like, rectal administration using creams, suppositories, or the like, transdermal administration using patches, liniments, gels, or the like,.

The administration route is preferably oral administration, nasal administration, or intravenous administration by injection.

The pharmaceutical composition according to the present invention may contain, in addition to R-ketamine or a pharmacologically acceptable salt thereof, another medical ingredient having a neuroprotective effect other than S-ketamine or a pharmacologically acceptable salt thereof.

In addition, the pharmaceutical composition may appropriately contain, in addition to these medical ingredients, a suitable pharmacologically acceptable carrier well known to those skilled in the art depending on the administration form and the like.

The pharmacologically acceptable carriers are not particularly limited, but examples thereof include an antioxidant, a stabilizer, a preservative, a flavoring agent, a coloring agent, a solubilizer, a solubilizing agent, a surfactant, a emulsifier, a defoamer, a viscosity adjuster, a gelling agent, an absorption enhancer, a dispersant, an excipient, a pH adjuster, and the like.

When the preventive or therapeutic agent, drug, and pharmaceutical composition for a neurodegenerative disease or a cognitive dysfunction according to the present invention are prepared as a formulation for injection, a formulation with the form of a solution or a suspension is preferable, when they are prepared as a formulation for nasal or oral transmucosal administration, a formulation with the form of a powder, a drop, or an aerosol agent is preferable, and when they are prepared as a formulation for rectal administration, a formulation with the form of a semisolid formulation such as a cream or a suppository is preferable.

Any of these formulations may be prepared by any method known to those skilled in the pharmaceutical arts, for example, the methods described in Remington's Pharmaceutical Sciences (Mac Publishing Company, Easton, PA, 1970).

For the formulation for injection, as examples of the carrier, plasma-derived proteins such as albumin, amino acids such as glycine, and sugars such as mannitol, may be used, and furthermore, buffers, solubilizing aid, isotonic agents, and the like may be used. In addition, when it is used as a water-soluble formulation or a freeze-dried formulation, for example, surfactants such as Tween (registered trademark) 80 and Tween (registered trademark) 20 may be used to prevent aggregation.

For the formulation for parenteral administration other than the formulation for injection, as examples of the carrier, distilled water or physiological saline, a polyalkylene glycol such as polyethylene glycol, oils of vegetable origin, hydrogenated naphthalene, and the like may be used. For example, the formulation for rectal administration such as a suppository may use, as excipients, for example, a polyalkylene glycol, vaseline, cacao oil, and the like. For the formulation for vaginal administration, as examples of the carrier, absorption enhancers such as bile salts, ethylenediamine salts, and citrates may be used. The formulations for inhalation may be solid, and as excipients, for example, lactose may be used. The drops for nasal administration may be a water or oil solution.

The exact dosage and administration plan of the preventive or therapeutic agent, drug, and pharmaceutical composition for a neurodegenerative disease or a cognitive dysfunction according to the present invention are adjusted depending on the required amount, the treatment method, the disease, the degree of need, and the like for each individual subject.

The dosage may be determined depending specifically on the age, the body weight, the general health state, the gender, the diet, the administration time, the administration method, the excretion rate, the drug combination, the medical condition of the patient, and the like. Furthermore, the dosage may be determined in consideration of other factors.

When the preventive or therapeutic agent, drug, and pharmaceutical composition for a neurodegenerative disease or a cognitive dysfunction according to the present invention are administered to a patient having a neurodegenerative disease or a cognitive dysfunction, R-ketamine or a pharmacologically acceptable salt thereof is preferably contained in an amount effective to reduce the symptom of each neurodegenerative disease or cognitive dysfunction, preferably the loss of neurons of each neurodegenerative disease or cognitive dysfunction.

R-ketamine or a pharmacologically acceptable salt thereof may be safely used because it has few of the side effects observed with S-ketamine or RS-ketamine, and the daily dosage differs depending on the condition and weight of the patient, the type of compound, the administration route, and the like. In the case of the parenteral administration, it is usually about 0.01 to 1000 mg/person/day, and it is preferably 0.1 to 500 mg/person/day. In the case of the oral administration, it is usually about 0.01 to 500 mg/person/day, and it is preferably 0.1 to 100 mg/person/day.

The present invention may be a method for preventing or treating a neurodegenerative disease or a cognitive dysfunction, which includes administering the compound I or a pharmacologically acceptable salt thereof to a patient in need thereof.

The patient in need thereof is not particularly limited, the patient is a mammal, and the patient is preferably a human.

The compound I or a pharmacologically acceptable salt thereof is preferably administered in a therapeutically effective amount.

In the present invention, since the compound I or a pharmacologically acceptable salt thereof has a neuronal loss alleviating effect, by administering before the onset of a neurodegenerative disease or a cognitive dysfunction, it may be used for the prevention of the neurodegenerative disease or the cognitive dysfunction, or it may be used for the treatment of the neurodegenerative disease or the cognitive dysfunction by the neuronal loss alleviating effect for each neurodegenerative disease or cognitive dysfunction.

### [Examples]

Hereinafter, the present invention will be described more specifically with reference to examples, but the present invention is not limited to the following examples. In addition, various changes can be made without departing from the technical concept of the present invention.

All tests were carried out with the permission of the Chiba University Animal Experiment Committee.

A Parkinson's disease model animal (Jackson-Lewis V. et al. Protocol for the MPTP mouse model of Parkinson's disease. Nat Protoc. 2007; 2 (1): 141-51.), which is a representative Parkinson's disease animal model and has been used by a number of research groups so far, was used to study the therapeutic effect of R-ketamine and S-ketamine on the loss of the dopamine nervous system in the brain of the model animal.

R-ketamine hydrochloride and S-ketamine hydrochloride were prepared from RS-ketamine (Ketalar (registered trademark), ketamine hydrochloride, Daiichi Sankyo Co., Ltd., Tokyo, Japan) using D-tartaric acid or L-tartaric acid by the method described in the specification of the U.S. Patent No. 6040479. The purity of each isomer was confirmed by high performance liquid chromatography (CHIRALPAK (registered trademark) IA, column size: 250 x 4.6 mm, mobile phase: n-hexane/dichloromethane/diethylamine (75/25/0.1), the retention time of S-ketamine = 6.99 min, and the retention time of R-ketamine = 10.56 min, Daicel Corporation, Tokyo, Japan).

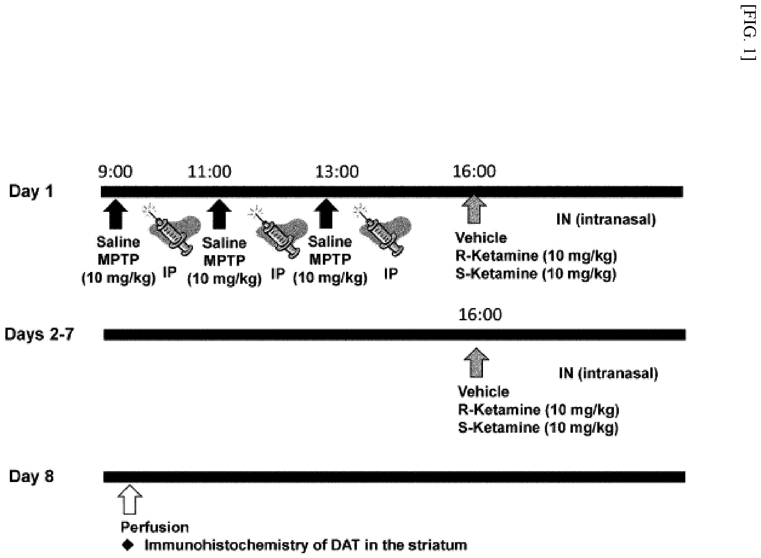

The creation of the Parkinson's disease model animal and the administration of the drug were specifically carried out as described below (FIG. 1). As the mouse, a male C57BL/6N mouse (12 weeks of age, SLC Japan Co., Ltd., Hamamatsu, Japan) was purchased. Mice had free access to water and food. For the experiment, those above 14 weeks of age were used. On the first day of the experiment, physiological saline (5 mL/kg body weight) or MPTP (10 mg/kg body weight) was intraperitoneally administered three times at two hours intervals. Three hours after the final administration, R-ketamine (10 mg/kg body weight) or S-ketamine (10 mg/kg body weight), or a vehicle (physiological saline 0.5 mL/kg) was intranasally administered. Similarly, from the second day to the seventh day of the experiment, R-ketamine (10 mg/kg body weight) or S-ketamine (10 mg/kg body weight), or the vehicle (physiological saline 0.5 mL/kg) was intranasally administered. (A) is a group administered with physiological saline + physiological saline, (B) is a group administered with MPTP + physiological saline, (C) is a group administered with MPTP + R-ketamine, and (D) is a group administered with MPTP + S-ketamine.

On the eighth day of the experiment, mice were perfused and fixed, and immunohistochemistry of dopamine transporter (DAT) was carried out. The immunohistochemistry of DAT was photographed with a microscope equipped with a CCD camera (Olympus IX70, Tokyo, Japan), and the shading was analyzed using SCION IMAGE software. Representative immunohistochemical photographs were taken with a Keyence microscope (BZ-9000, Osaka, Japan).

Statistical analysis was carried out by one-way analysis of variance (one-way ANOVA) followed by Tukey's test (Tukey HSD test). Data are expressed as mean ± standard error (n = 7 to 8 mouse/group). * p < 0.05, ** p < 0.01, and *** p < 0.001 show significant differences compared to the (B) group of mice treated with MPTP + physiological saline.

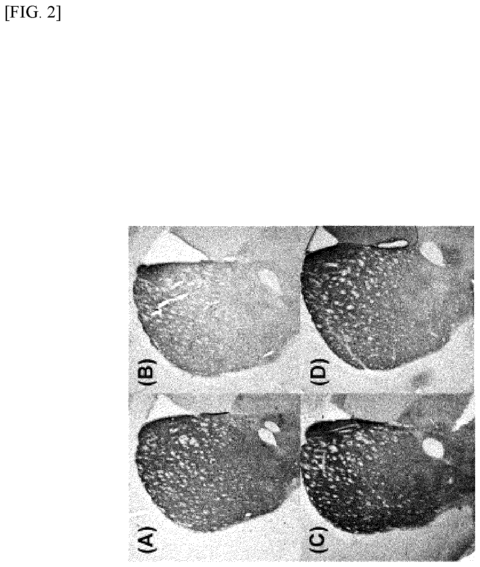

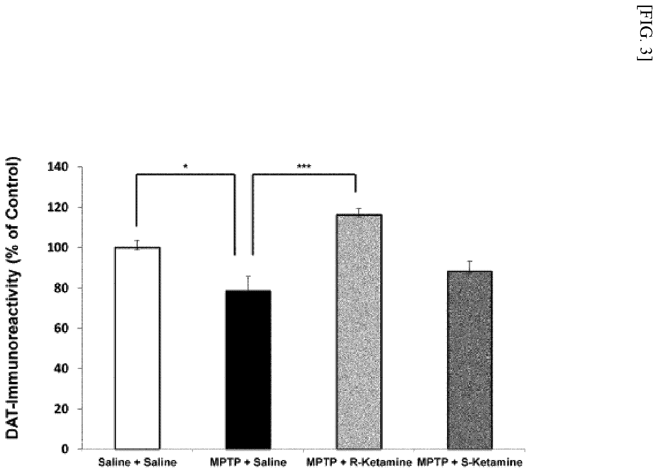

In mice of the group (B) in which MPTP was administered to male mice, DAT density in the striatum was significantly decreased as compared with normal mice of the group (A) in which physiological saline was administered. In the group (C), intranasal administration of R-ketamine significantly improved the decrease in DAT density in the striatum due to MPTP administration. On the other hand, in the group (D), intranasal administration of S-ketamine did not improve the decrease in DAT density in the striatum (FIG. 2 and FIG. 3).

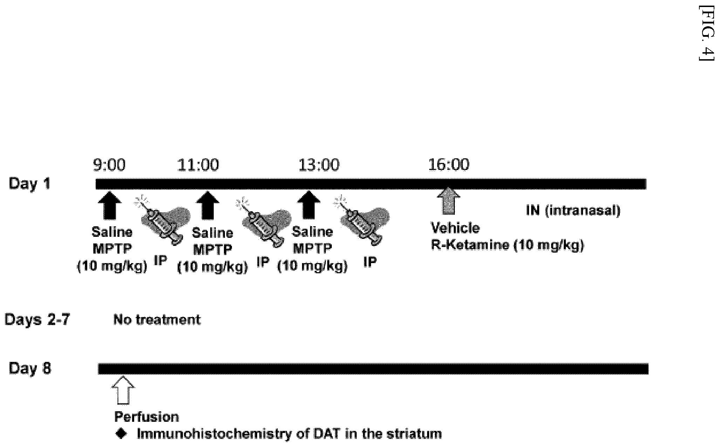

The test was carried out in the same manner as in the test in FIG. 1 except that R-ketamine (10 mg/kg body weight) or the vehicle (physiological saline 0.5 mL/kg) was intranasally administered once (FIG. 4).

There were a group administered with physiological saline + physiological saline, a group administered with MPTP + physiological saline, and a group administered with MPTP + R-ketamine.

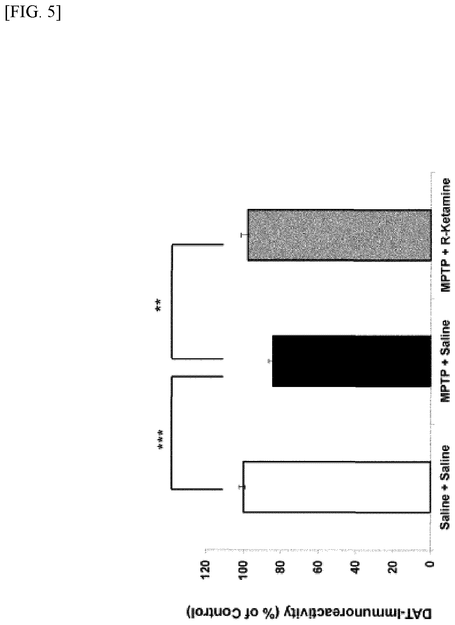

R-ketamine suppressed MPTP-induced decrease in DAT density in the striatum even with a single administration (FIG. 5).

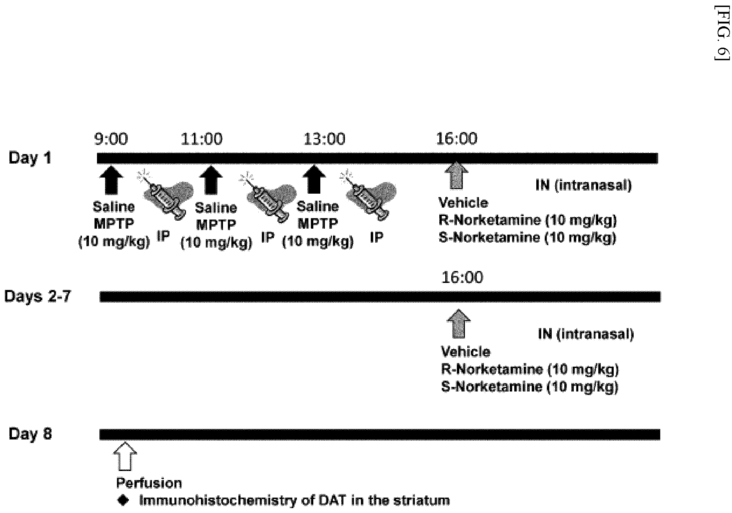

The test was carried out in the same manner as in the test in FIG. 1 except that R-norketamine (10 mg/kg body weight) or S-norketamine (10 mg/kg body weight) was administered (FIG. 6).

There were a group administered with physiological saline + physiological saline, a group administered with MPTP + physiological saline, a group administered with MPTP + R-norketamine, and a group administered with MPTP + S-norketamine.

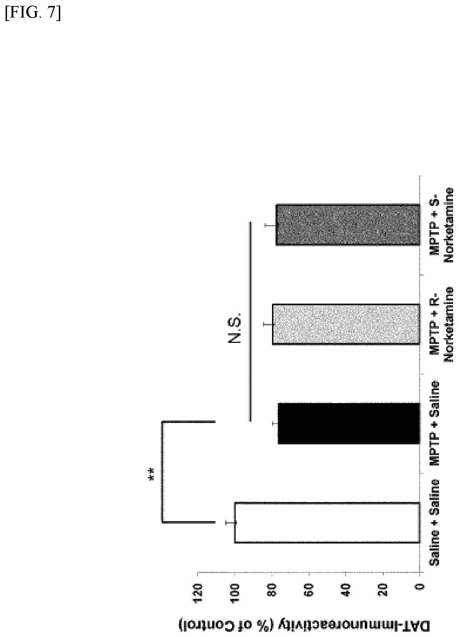

Norketamine is a metabolite of ketamine, but R-norketamine and S-norketamine did not suppress MPTP-induced decrease in DAT density in the striatum (FIG. 7).

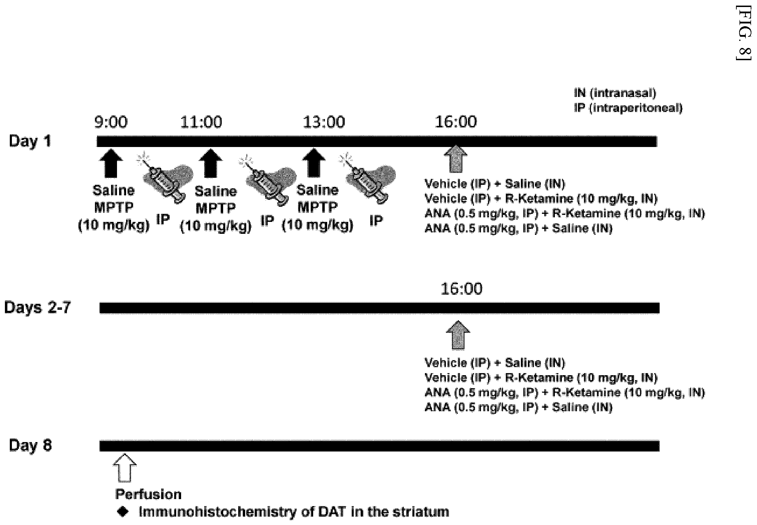

The test was carried out in the same manner as in the test in FIG. 1 except that ANA-12 (0.5 mg/kg), an antagonist of the TrkB receptor for brain-derived neurotrophic factor (BDNF: Brain-derived Neurotrophic Factor), was administered before the administration of R-ketamine. (FIG. 8). It should be noted that in FIG. 8, ANA-12 is described as ANA.

There was a group administered with physiological saline + physiological saline, a group administered with MPTP + physiological saline, a group administered with MPTP + R-ketamine, a group administered with MPTP + R-ketamine + ANA-12, and a group administered with MPTP + ANA-12.

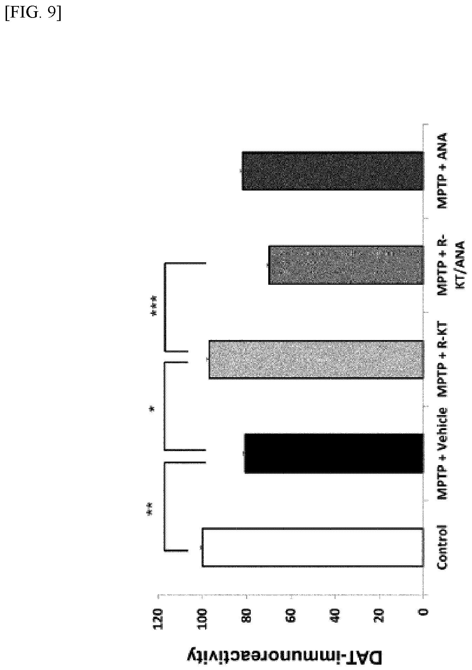

No effect of R-ketamine was observed by administration of ANA-12 (FIG. 9). On the other hand, administration of ANA-12 alone did not affect MPTP-induced decrease in DAT density (FIG. 9).

The above results revealed that intranasal administration of R-ketamine at a dose of 10 mg/kg showed a therapeutic effect in the MPTP-treated mice, which were an animal model of Parkinson's disease. On the other hand, intranasal administration of S-ketamine at a dose of 10 mg/kg showed no therapeutic effect in the MPTP-treated mice, which were an animal model of Parkinson's disease. Since the pharmacokinetics of both isomers of ketamine were the same, it was not considered to be due to differences in pharmacokinetics.

In addition, since R-norketamine, which is the main metabolite of R-ketamine, did not show a therapeutic effect, it was found that the effect after the administration of R-ketamine was not caused by the metabolite but by R-ketamine itself, which is the active substance. More interestingly, R-ketamine showed a therapeutic effect even with a single administration.

From the results of the present example, it was confirmed that S-ketamine had no effect and R-ketamine had an effect. Since S-ketamine has a higher affinity for NMDA receptors, it was considered that the therapeutic effect of R-ketamine in the MPTP-treated mice, which were an animal model of Parkinson's disease, was due to a mechanism other than the NMDA receptor blocking effect.

Furthermore, the results of the present example, in which the TrkB antagonist ANA-12 was pre-administered to investigate the mechanism of action of R-ketamine, suggested that the TrkB receptor was involved in the therapeutic effect of R-ketamine.

Using an animal model of Alzheimer's disease (intracerebroventricular administration of β-amyloid), which has been used by a number of research groups so far, the therapeutic effect of R-ketamine and S-ketamine on the cognitive dysfunction was studied.

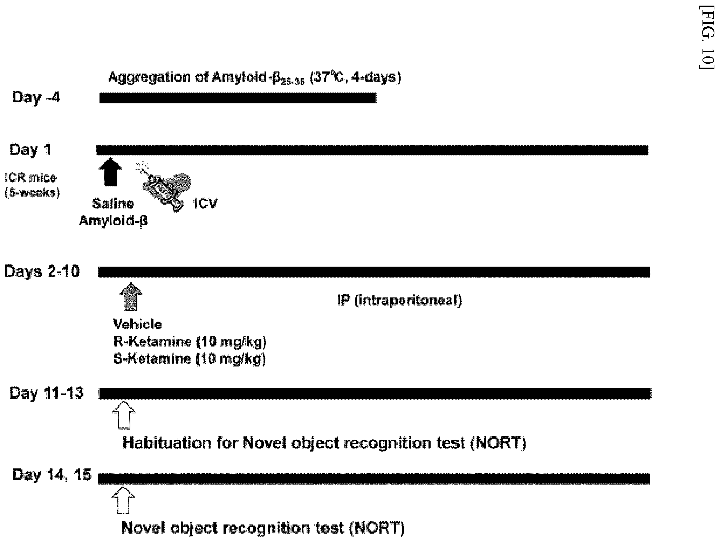

The creation of the Alzheimer's disease model animal and the administration of the drug were specifically carried out as described below (FIG. 10). As the mouse, a male ICR mouse (five weeks of age, SLC Japan Co., Ltd., Hamamatsu, Japan) was purchased. Mice had free access to water and food. First, β-amyloid (BACHEM, Cat. #: H-1 192.0005) was incubated at 37°C for four days to let aggregate. On the first day of the experiment, mice were anesthetized with isoflurane and pentobarbital, and placed on a mouse brain fixation apparatus. Distilled water (6 µL) or β-amyloid (6 µg) was administered intracerebroventricularly. On the next day (24 hours after the last administration), R-ketamine (10 mg/kg body weight) or S-ketamine (10 mg/kg body weight) or a vehicle (physiological saline 10 mL/kg) was intraperitoneally administered. Similarly, until the 10^{th} day of the experiment, R-ketamine (10 mg/kg body weight) or S-ketamine (10 mg/kg body weight) or the vehicle (physiological saline 10 mL/kg) was intraperitoneally administered once a day (FIG. 10). Administration was not made after the 11^{th} day of the experiment.
The novel object recognition test (NORT: Novel Object Recognition Test) is a system used for evaluating cognitive functions. NORT was carried out in accordance with the previous report (Hashimoto et al. Eur. J. Pharmacol. 2005; 519 (1-2): 114-117). Acclimation with a NORT apparatus (50.8 × 50.8 × 25.4 cm) was carried out for 10 minutes a day from the 11^{th} to the 13^{th} day of the experiment. On the 14^{th} day of the experiment, two objects having different shapes and colors were placed on the NORT apparatus at a distance of 35.5 cm, and the time for searching for each object was measured. A training session (10 minutes) was carried out, and a test session (5 minutes) was carried out on the following day, the 15^{th} day of the experiment (FIG. 10). In the test session on the next day, one object was changed to another new object (novel object). The time to search for two objects was measured, and the ratio of the time to search for a new object to the time to search for the two objects in the test session on the second day was calculated.

Statistical analysis was carried out by one-way analysis of variance (one-way ANOVA) followed by the Fisher's least significant difference test (Fisher's Least Significant Difference test). Data are expressed as mean ± standard error (n = 5 to 9 mouse/group). ** p < 0.01 and *** p < 0.001 show significant differences compared to the group of mice treated with β-amyloid + physiological saline.

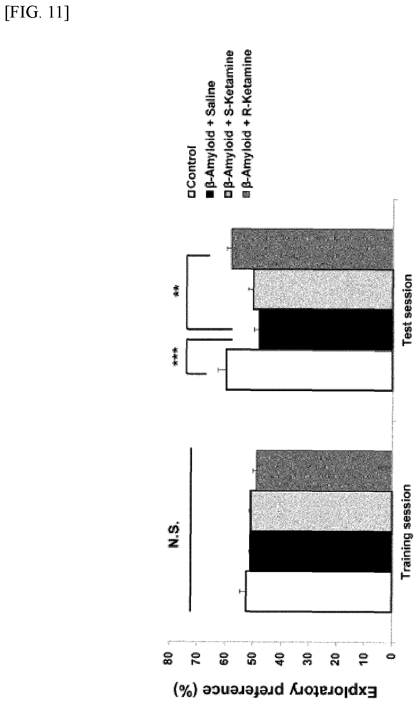

Significant decrease in cognitive function was observed in the male mice treated with β-amyloid. Intraperitoneal administration of R-ketamine significantly improved the decrease in cognitive function caused by β-amyloid administration. On the other hand, intraperitoneal administration of S-ketamine did not improve the decrease in cognitive function caused by β-amyloid administration (FIG. 11).

The above results revealed that intraperitoneal administration of R-ketamine at a dose of 10 mg/kg showed a therapeutic effect in the β-amyloid-treated mice, which were an animal model of Alzheimer's disease. However, intraperitoneal administration of S-ketamine at a dose of 10 mg/kg did not show a therapeutic effect in the β-amyloid treated mice, which were an animal model of Alzheimer's disease. Since the pharmacokinetics of both isomers of ketamine were the same, it was not considered to be due to differences in pharmacokinetics.

In addition, from the results of the present example, it was confirmed that S-ketamine had no effect and R-ketamine had an effect. Since S-ketamine has a higher affinity for NMDA receptors, it was considered that the therapeutic effect of R-ketamine in the β-amyloid-treated mice, which was an animal model of Alzheimer's disease, was due to a mechanism other than the NMDA receptor blocking effect.

The therapeutic effects of R-ketamine and S-ketamine on cognitive dysfunction were studied using animal models of Lewy body dementia and diseases caused by α-synuclein aggregation (intracerebroventricular administration of α-synuclein), which have been used by a number of research groups so far.

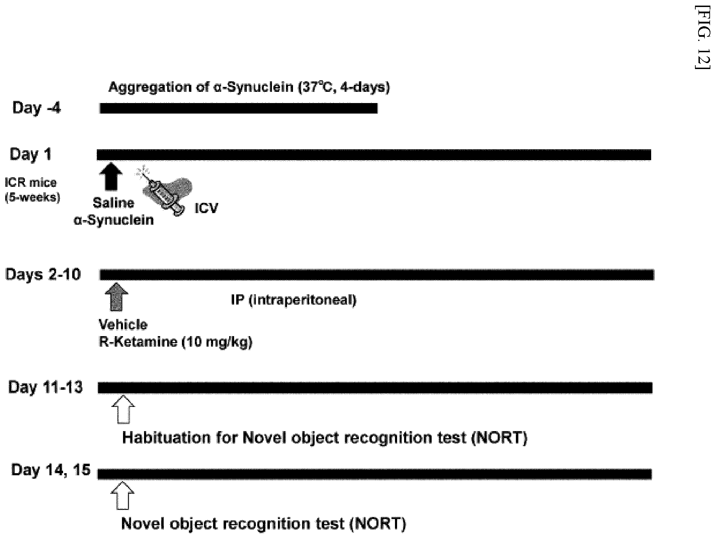

The creation of the Lewy body dementia model animal and the administration of the drugs were specifically carried out as described below (FIG. 12). As the mouse, a male ICR mouse (five weeks of age, SLC Japan Co., Ltd., Hamamatsu, Japan) was purchased. Mice had free access to water and food. First, α-synuclein (StressMarq @ Bioscience, Cat #: SPR-322) was incubated at 37°C for four days to let aggregate. On the first day of the experiment, mice were anesthetized with isoflurane and pentobarbital, and placed on a mouse brain fixation apparatus. Physiological saline (6 µL) or α-synuclein (6 µg) was administered intracerebroventricularly. On the next day (24 hours after the last administration), R-ketamine (10 mg/kg body weight) or a vehicle (physiological saline 10 mL/kg) was intraperitoneally administered. Similarly, until the 10^{th} day of the experiment, R-ketamine (10 mg/kg body weight) or the vehicle (physiological saline 10 mL/kg) was intraperitoneally administered once a day (FIG. 12). Administration was not made after the 11^{th} day of the experiment.

NORT was carried out in the same manner as described above. Acclimation with a NORT apparatus was carried out for 10 minutes a day from the 11^{th} to the 13^{th} day of the experiment. On the 14^{th} day of the experiment, a training session was carried out, and a test session (5 minutes) was carried out on the following day, the 15^{th} day of the experiment (FIG. 12).

Statistical analysis was carried out by one-way analysis of variance (one-way ANOVA) followed by the Fisher's least significant difference test (Fisher's Least Significant Difference test). Data are expressed as mean ± standard error (n = 4 mouse/group). * p < 0.05 and ** p < 0.01 show significant differences compared to the group of mice treated with α-synuclein + physiological saline.

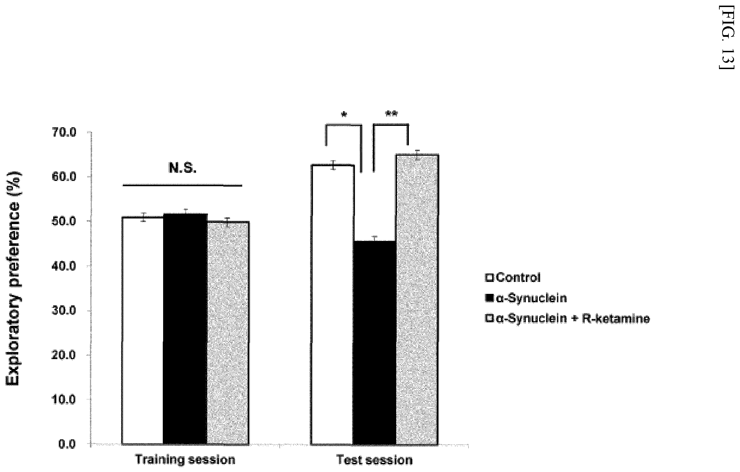

Significant decrease in cognitive function was observed in the male mice treated with α-synuclein. Intraperitoneal administration of R-ketamine significantly improved the decrease in cognitive function caused by α-synuclein administration (FIG. 13).

The above results revealed that intraperitoneal administration of R-ketamine at a dose of 10 mg/kg showed a therapeutic effect in the α-synuclein-treated mice, which were animal models of Lewy body dementia and diseases caused by aggregation of α-synuclein. In addition, from the results of the present example, R-ketamine is useful as a preventive or therapeutic drug for Lewy body dementia and diseases caused by aggregation of α-synuclein.

### [Industrial applicability]

In the present invention, a preventive or therapeutic agent for a neurodegenerative disease or a cognitive dysfunction containing the compound I, preferably R-ketamine or a pharmacologically acceptable salt thereof, as an active ingredient is useful as a novel pharmaceutical.

## Claims

1. A preventive or therapeutic agent for a neurodegenerative disease or a cognitive dysfunction, comprising a compound represented by a following formula (I) or a pharmacologically acceptable salt thereof as an active ingredient.
Formula (I): 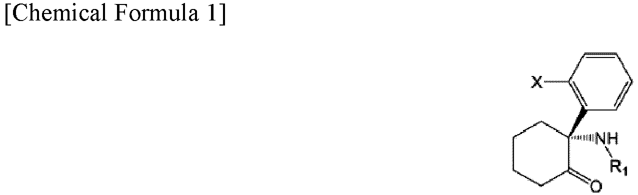 (In the formula (I),
X is H, alkyl, alkenyl, aryl, F, Cl, Br, or I; and
Ri is alkyl, alkenyl, or aryl.)

2. The preventive or therapeutic agent for a neurodegenerative disease or a cognitive dysfunction according to claim 1, comprising R-ketamine or a pharmacologically acceptable salt thereof.

3. The preventive or therapeutic agent for a neurodegenerative disease or a cognitive dysfunction according to claim 1 or 2, wherein the neurodegenerative disease is Parkinson's disease, Parkinson's syndrome, a disease that shows chorea movement, spinal cord cerebellar degeneration, amyotrophic lateral sclerosis, spinal muscular atrophy, primary lateral sclerosis, spinal and bulbar muscular atrophy, syringomyelia, neurospinous erythrocytosis, eating disorders, substance use disorders (drug dependency), Alzheimer's disease, Lewy body dementia, basal ganglia degeneration, multiple sclerosis, or the like or is a neurodegenerative disease based on traumatic brain injury, cerebral infarction, or cardiovascular disease.

4. The preventive or therapeutic agent for a neurodegenerative disease or a cognitive dysfunction according to claim 1 or 2, wherein the cognitive dysfunction is a cognitive dysfunction based on a neurodegenerative disease or a cognitive dysfunction based on a psychiatric disease comprising depression, bipolar disorder, anxiety disorder, and autism, a circulatory disease, and a medical disease comprising cancer.

5. The preventive or therapeutic agent for a neurodegenerative disease or a cognitive dysfunction according to any one of claims 1 to 4, not substantially comprising a compound represented by a following formula (II) or a pharmacologically acceptable salt thereof.
Formula (II): 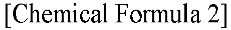 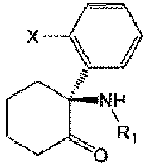 (In the formula (II),
the definitions of X and Ri are the same as the definitions in the formula (I).)

6. The preventive or therapeutic agent for a neurodegenerative disease or a cognitive dysfunction according to any one of claims 1 to 5, not substantially comprising S-ketamine or a pharmacologically acceptable salt thereof.

7. A pharmaceutical composition comprising the preventive or therapeutic agent for a neurodegenerative disease or a cognitive dysfunction according to any one of claims 1 to 6.
